# EUROPEAN PATENT SPECIFICATION

(11) **EP 2 352 421 B1**
(45) Date of publication and mention of the grant of the patent: **10.10.2018**
(21) Application number: 09825578.9
(22) Date of filing: 10.11.2009
(51) Int. Cl.: A61B 5/00, A61B 5/04, A61B 90/00, A61B 5/0408, A61B 5/0428

(54) **SENSOR ARRAY SYSTEM AND ASSOCIATED METHOD OF USING SAME**
SENSORENARRAYSYSTEM UND ENTSPRECHENDES VERFAHREN ZU SEINER ANWENDUNG
SYSTÈME DE RÉSEAU DE CAPTEURS ET PROCÉDÉ ASSOCIÉ POUR L'UTILISER

(30) Priority: 10.11.2008 US 112939 P
(43) Date of publication of application: 10.08.2011
(73) Proprietor: Cardioinsight Technologies, Inc., Cleveland, OH 44106-3052 (US)
(72) Inventor: RAMANATHAN, Charulatha, Solon OH 44139 (US); WODLINGER, Harold M., Thornhill ON L4J 1B9 (CA); PING, Jia, Solon OH 44139 (US); ARLESS, Steven G., Baie Durfe QC H9X 2V1 (CA); HARRIS, Gasparakis, St. Louis MO 63108 (US)
(74) Representative: Vigand, Philippe
(86) International application number: PCT/US2009/063803
(87) International publication number: WO 2010/054352

(56) References cited:
- EP-A2- 0 509 689
- EP-A2- 0 571 040
- CN-Y- 2 683 026
- US-A- 3 998 213
- US-A- 5 327 888
- US-A1- 2003 065 253
- US-A1- 2008 154 110
- US-A1- 2008 249 389

## Description

### CROSS-REFERENCE TO RELATED APPLICATION

### TECHNICAL FIELD

This invention relates to a system and method for sensing electrical activity of a patient, and more particularly to a sensor array and methods of using a sensor array to acquire electrical data.

### BACKGROUND

Various electrophysiology techniques have been developed for collecting electrophysiology data for a patient. Invasive measurement techniques typically involve placing one or more electrodes into contact with biological tissue. For example, an electrophysiology catheter or probe contains one or more electrodes at its distal end, each electrode being able to record electrical activity at the location of said electrode. Thus, by placing the catheter at a particular location relative to a patient's organ, such as the heart, organ-specific electrical activity can be recorded. Sensors can also be arranged on a body surface of a patient for non-invasive acquisition of electrical information. Signal processing, such as filtering, can be applied on any such signal to remove noise or otherwise enhance the recorded activity. The document US2008/249389 discloses a sensor array that includes multiple separable branches and a pad having an electrode disposed thereon attached to a distal end of the branch 20. As illustrated in FIG. 8 and disclosed in paragraph [0040] of this document, when the set is placed on the patient, the core multiple branches are separated from each other and the electrodes are strategically placed on the patient. Further art is disclosed by the document US 5,327,888 which describes a single precodial array. The array is comprised of a flexible strip. The document EP 509 689 discloses an electrode strip that includes an elongate substrate divided into five elongate sections. Conductors provide a connection to electrodes disposed at the end of each section. The document US 3,998,213 discloses a self-adjustable electrode holder that can be positioned on a head a patient to measure electrical brain activity. The holder includes multiple straps connected in a hemispherical pattern. The document EP 571 040 discloses an electrocardiography electrode positioning device or sling that is worn on a bust of a patient. The device includes a pectoral part including opposite extending tabs having electrodes disposed thereon. An inguinal element is connected to the pectoral part via straps and includes a pair of electrodes.

The present invention concerns a sensor array system as defined in the appended claims.

### BRIEF DESCRIPTION OF THE DRAWINGS

FIG. 1 depicts an example of a sensor array system according to an aspect of the invention.
FIG. 2 depicts part of a sensor array section demonstrating electrically conductive portions thereof.
FIG. 3 depicts an enlarged view of part of the front sensor array section from FIG. 1.
FIG. 4 depicts a sensor array section applied to a front torso of a patient.
FIG. 5 depicts a front sensor array section applied to a front torso of a patient with a portion of the sensors repositioned.
FIG. 6 depicts an enlarged view of a right sensor array section from FIG. 1.
FIG. 7 depicts a sensor array section applied to a rear torso of a patient.
FIG. 8A depicts an example of a first layer of a sensor that can be utilized in a sensor array according to an aspect of the invention.
FIG. 8B depicts an example of a second layer of a sensor that can be utilized in a sensor array according to an aspect of the invention.
FIG. 8C depicts an example of a third layer of a sensor that can be utilized in a sensor array according to an aspect of the invention.
FIG. 8D depicts an example of a fourth layer of a sensor that can be utilized in a sensor array according to an aspect of the invention.
FIG. 9 is a side view of an elongated section of a sensor array demonstrating a multi-layered structure thereof.
FIG. 10A is a top view of a first example layer from the structure of FIG. 9.
FIG. 10B is a top view of a second example layer from the structure of FIG. 9.
FIG. 10C is a top view of a third example layer from the structure of FIG. 9.
FIG. 10D is a top view of a fourth example layer from the structure of FIG. 9.
FIG. 11A is a schematic illustration demonstrating use of part of a sensor array alignment mechanism.
FIG. 11B is a schematic illustration demonstrating a marking layer to facilitate alignment and positioning of a sensor array on a patient.
FIG. 11C is a schematic illustration demonstrating a sensor array section applied over the markings depicted in FIG. 11B.
FIG. 12 depicts an example of a data acquisition system according to an aspect of the invention.
FIG. 13 depicts an example of a graphical user interface that can be used in the data acquisition system of FIG. 12.
FIG. 14 depicts an example of another graphical user interface that can be provided in connection with the data acquisition system of FIG. 12.
FIG. 15 depicts an example of an imaging system that can be utilized to generate geometry data for a patient for use with a sensor system according to an aspect of the invention.

### SUMMARY

Systems and methods are disclosed for sensing electrical activity of a patient.

According to one embodiment, a sensor array system includes a plurality of elongated strips of a flexible substrate material that extend from a first end to terminate in a second end spaced therefrom by a length of the substrate material having spaced apart side edges. A plurality of electrically conductive sensors are connected with and distributed along the length of each of the plurality of elongated strips to provide a column of sensors along each respective strip. The strips can be connected via connecting elements to maintain a spatial arrangement of the elongated strips. Electrically conductive paths extend from each of the conductive sensors through a respective strip to which it is connected and terminate in a corresponding connector located near the first end of the respective strip.

According to another embodiment, the sensor array system can include a plurality of sensor array sections, each being configured for placement over a selected region of a patient's torso.

According to yet another embodiment, the sensor array system can be utilized to acquire electrical signals from the plurality of sensors positioned on a body surface of the patient.

### DETAILED DESCRIPTION

The invention relates generally to a system and method for sensing electrical activity of a patient, and more particularly to a sensor array assembly and methods of using same to acquire electrical data.

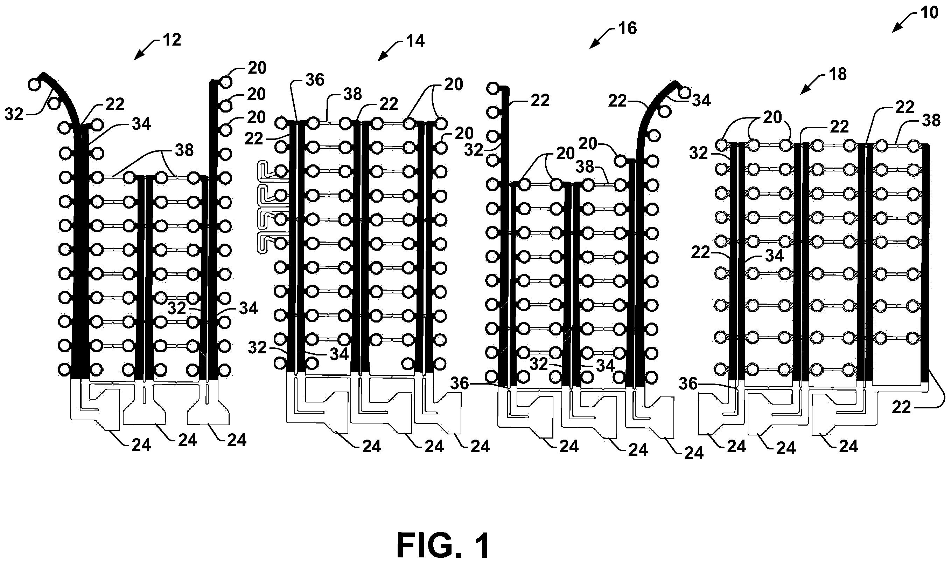

FIG. 1 depicts an example of a sensor array system 10 according to an aspect of the invention. The example sensor array system 10 includes a plurality sensor array sections 12, 14, 16, and 18. Each of the sections 12, 14, 16 and 18 is dimensioned and configured for placement overlying a different anatomical region of a patient's body. The sections 12, 14, 16 and 18 are in the form of flexible or pliant sheets conform able to the curvature of a patient's anatomy to which they are placed. Each of the sections 12, 14, 16 and 18 includes a plurality of sensors 20 arranged in a generally evenly distributed relationship according to the anatomical regional over which it is to be positioned. Different sections 12, 14, 16 and 18 can have different numbers and distributions of sensors 20 depending on where the respective sections are to be positioned.

In the example of FIG. 1, sections 12 and 16 are configured for placement along the lateral (or side) portions of a patient's torso. The section 14 can be placed on the front side of a patient's torso, and section 18 can be utilized for placement on the back side of a patient's torso. Thus, the sections 12, 14, 16 and 18 can be utilized to cover a patient's entire torso with a plurality of generally evenly spaced apart sensors 20. When the sensor array sections 12, 14, 16 and 18 are positioned on the patient's torso, they provide a generally evenly distributed arrangement of sensors that can cover and completely circumscribe the patient's torso.

While in example FIG. 1, four separate sensor array sections 12, 14, 16 and 18 are illustrated, it is to be understood and appreciated that a fewer or greater number of sensor sections can be utilized to form a sensor array system 10 according to an aspect of the invention. For example the sensor array system 10 could be formed of individual strips 22 (or individual strip portions 32 and 34). Alternatively some or all of the sections 12, 14, 16 and 18 could be connected together.

Each of the sections 12, 14, 16 and 18 also includes one or more strips 22 of a flexible substrate material to which each of the sensors 20 is attached. The strips 22 can be substantially linear elongated sheets of the substrate material that extend between spaced apart ends thereof. The length of each strip 22 can also vary according to the anatomical region on which it is to be placed. Alternatively or additionally, some strips can include curved portions, such as end strips in sections 12 and 16. For instance, the distribution of sensors in each section can be in the form of a two dimensional matrix of sensors arranged in columns along the length of each of the strips 22 (*e.g.,* seen from top to bottom in the view of FIG. 1) and rows (*e.g.,* seen from left to right in the view of FIG. 1).

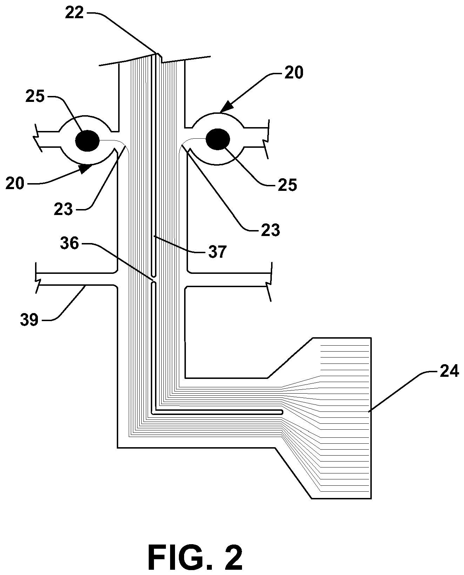

Referring to FIG. 2, each of the strips 22 carries a set of electrically conductive elements (*e.g.*, wires or traces) 23 that provide for communication of sensed electrical signals from each of the respective sensors 20 to corresponding connectors 24 located at a proximal end of the respective strips 22. Each of the conductive elements 23 provides an electrically conductive path that extends from an electrically conductive sensor body portion 25 each of the sensors 20 to the strip and travels along the strip where it terminates at a respective connector 24. Each of the connectors 24 can be electrically connected to a connector box (not shown), which can be used to communicate the electrical signal information to a data acquisition system for further processing. Each of the connectors 24 thus is configured to gather together electrical traces or wires from sensors disposed on one or more strip 22. Each of the sensor array sections 12, 14, 16 and 18 can have electrically conductive paths similar to that shown and described in the example of FIG. 2.

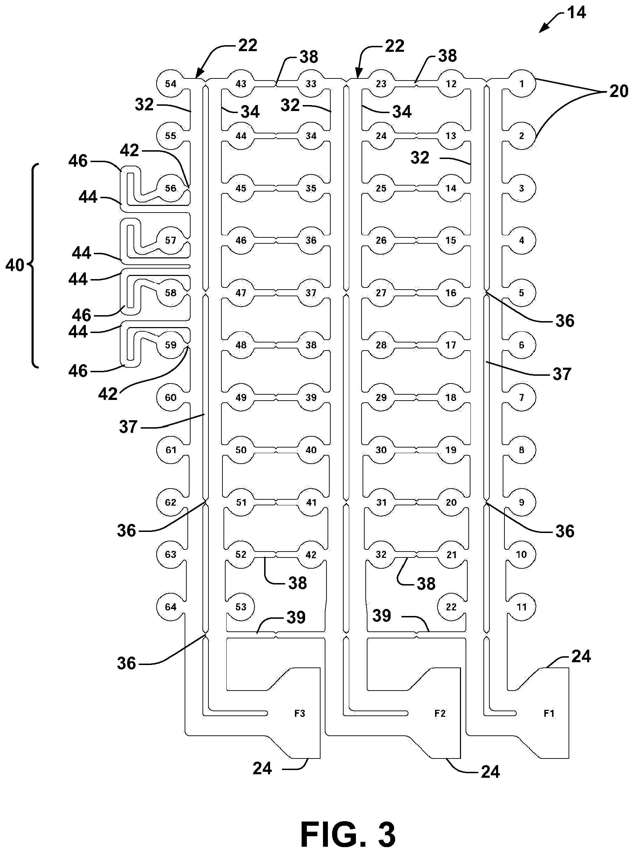

The attachment of the sensors 20 via the substrate material and conductors 23 to the respective strips 22 maintains a desired relative position of the sensors. As described herein, however, one or more of the sensor array sections can be configured to allow repositioning of sensors 20. The repositioning can be enabled by providing a set of one or more repositionable sensor (See, *e.g.,* FIGS. 3-5). Alternatively or additionally, portions of one or more strips 22 can be moveable relative other strips in a given sensor array section such as for selective positioning of combinations of sensors and strips.

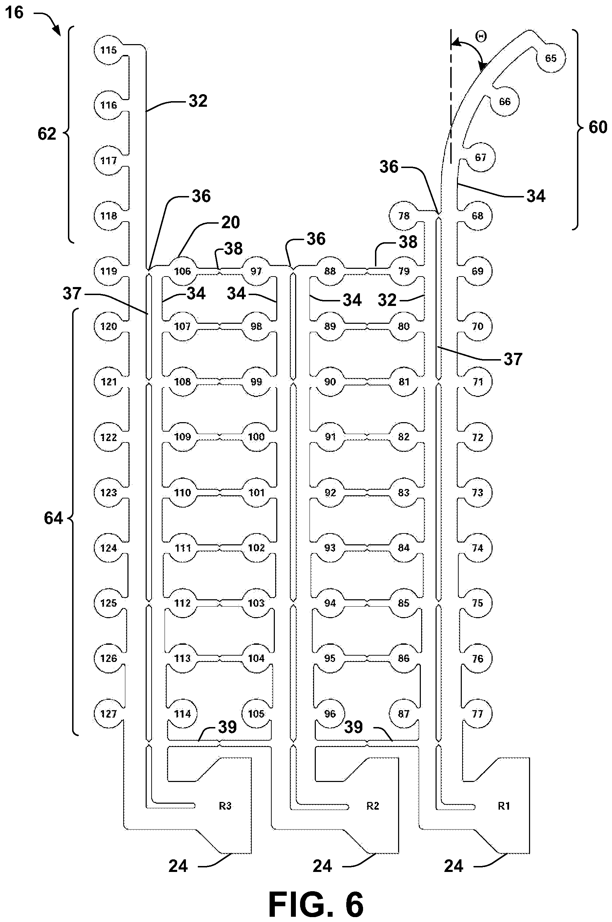

For example, as better demonstrated in 22 the enlarged views of FIGS. 3 and 6, each strip 22 (or at least some of the strips) can be formed of a pair of generally parallel strip portions 32 and 34 that are connected together along an adjacent edge thereof by connecting elements (or tabs) 36, which forms spatial voids 37 between adjacent connecting elements. The connecting elements 36 can be distributed at spaced apart locations along the coextensive length of each strip pair 32 and 34. Thus, each strip portion 32 and 34 can itself be considered a separate strip of the flexible substrate material. For instance, in the examples of FIGS. 2, 3 and 5, connecting elements 36 are provided near ends of the strips 22 as well as at intermediate locations between the ends, such as with a spacing that is greater than the longitudinal spacing between sensors 20 on the respective strips. It will be appreciated that different spacing between connecting elements 36 could be utilized, which could be greater or lesser from that shown in the figures.

Each of the connecting elements 36 can be configured as having a frangible structure (*e.g.,* a tear seam, perforations or a reduced with) at an intermediate location that can be easily cut or torn. When one or more connecting element 36 is cut or torn, the adjoining strip portions 32 and 34 and their associated sensors 20 can be repositioned relative to each other. One or more connecting element 36 for a given strip 22 can be broken to enable a fractional length or the entire length of strip portion pair 32 and 34 to be repositioned relative to each other. For instance, the strip portions 32 and 34 in a given strip 22 can be separated from each other by a desired distance, such as to accommodate a larger patient.

Additionally or alternatively, adjacent pairs of sensors 20 that extend outwardly toward each other from an adjacent pair of strips 22 can be connected together by elongated connecting elements (or tabs) 38. Similar to the other connecting elements 36 extending between strip portions, the sensor connecting elements 38, support adjacent columns of sensors in a nominal configuration. An intermediate portion of each of the connecting elements 38 can be configured as including a frangible portion (*e.g.,* including perforations a tear seam or a reduced width), which can be easily torn or cut. Thus, by breaking the connecting element 38 between a pair of adjacent sensors, the respective sensors 20 and their associated strips 22 may be repositioned relative to each other in a different manner from the nominal configuration.

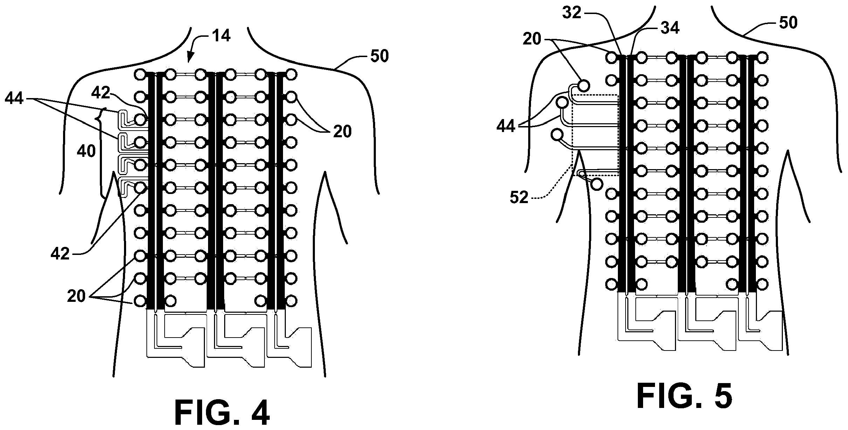

Additionally, other strip connecting elements 39 can be connected between strip portions 34 and 32 that feed conductors to different connectors. As shown in FIGS. 3 and 5, the strip connecting elements 39 are positioned between the strip portions 34 and 32 at a location between the connectors and where the sensors 20 begin along the strips 22. For example, the strip connecting elements 39 can be aligned in the same row as the first strip connecting element 36 that connect strip portions 32 and 34 of each given connector 24. Such connecting elements cooperate to maintain a desired orientation of the strips as the connecting elements are moved around (*e.g.,* to a connector box or other intermediate extension connectors).

Other features can be utilized to facilitate accommodating patients of varying size. For instance, the distance between adjacent sensors along the length of the strip can be extendable, such as by providing accordion-like folds between adjacent sensors. Similar accordion-like folds or a spiral length of substrate can also be provided between adjacent elongated strips (which provide the lateral extensions and tear seams). Thus, the portions of the strips on opposing sides of such folds or spirals can be separated apart from each other with ease or brought closer together according to the shape or size of the patient. The sensor array sections can also be provided in more than one size.

As a further example, selected sensors 20 and portions of the strips 22 may be removed (*e.g.,* by cutting) from a given section such as to accommodate smaller sized patients. Typically such removal is performed by cutting a length of a given strip 22 including extra sensors from a distal end of the strip that is opposite the end where connectors 24 are located. Such repositioning and/or removal of sensors 20 and strips enables the same set of strips to be utilized for a variety of different body types, body sizes and under different conditions.

The sensor array assembly 10, including the sensors 20 and strips 22, can be formed of radio-translucent materials. For example, the sensors 20 can be formed of a radio-translucent material, such as silver and/or silver chloride. Alternatively, the electrically conductive elements in the strips 22 can be formed of a thermal carbon fiber material, such as disclosed in U.S. Patent No. 5,523,534. Those skilled in the art will understand and appreciate other materials that can be utilized to provide desired radio-translucency of the sensor array system 10 and its constituent parts. The sensor array system 10 can be manufactured for single use only and be disposable. Alternatively, it may be manufactured and assembled to provide for more than one use.

Referring to FIG. 3, the front sensor array section 14 is depicted in an enlarged view to demonstrate more fully some additional features that can be implemented for this and other sensor array sections. As mentioned, the sensor array section 14 is configured as a generally rectangular sheet of flexible material that includes a plurality of sensors 20. In its normal, fully connected configuration (*e.g.*, no connecting elements being broken), the sensors 20 are arranged in a substantially evenly distributed two dimensional matrix of rows and columns. In the example of FIG. 3, each of the sensors 20 is numbered (*e.g.*, labeled '1' to '64'). Each of the sensors 20 in the other sections 12, 16 and 18 similarly could be numbered or otherwise uniquely indentified to facilitate identification of sensors via a user interface of a data acquisition system.

The connectors 24 can also be identified by a combination of a letter and a number that uniquely identifies each connector. For example, "F" can denote front section, 14 "R" can denote a right-side section 16, "L" can denote a left-side section 12, and "B" can denote a back section 18. Thus, in the example of FIG. 3, the connectors are labeled F1, F2 and F3, wherein connector F1 includes two columns of sensors identified as sensors labeled '1'-'22', connector F2 includes two columns of sensors labeled '23'-'42', and connector F3 includes two columns of sensors labeled as '43'-'64'.

Since the section 14 is intended for placement on a front side of a patient's torso, selected sensors 20 are configured to facilitate its use with a defibrillator and/or catheter mapping patch. For instance, a defibrillator patch may be positioned on the patient's torso in combination with use of the front sensor array section 14. To facilitate placement of these types of patches or pads, it may be required to either reposition or remove certain of the sensors 20 from the sensor array section 14. Thus, in the example of section 14 in FIG. 3, a set 40 of individually positionable or floating sensors (*e.g.*, sensors labeled '56', '57', '58' and '59') are configured to facilitate their individual positioning relative to the strip 22.

By way of further example, each sensor in the set 40 includes two attachment members that connect the respective sensors to the strip 22. A first attachment member 42 for each sensor includes a frangible section of a substrate material. The length of the first attachment 42 member is commensurate with the connection between other sensors 20 and the edge of the strip portion 32 from which the sensors extend. In contrast to the attachment of the other sensors, the first attachment member 42 for each floating sensor in the set 40 is not utilized for providing a signal path from the respective sensors to the connector 24. Instead, the first attachment member is utilized to maintain the sensors 20 in a desired nominal arrangement similar to the other sensors. Thus, the first attachment 42 can be broken (*e.g.,* cut or torn) without impairing the communication of sensed electrical signals from the sensor 20 to the connector 'F3'.

Each second attachment member 44 includes an elongated connecting member of a flexible material that extends from the strip portion 36 around a portion of the sensor 20 and connects to the sensor at diametrically opposite side from where the first attachment member connects to the sensor. To provide additional length, the second attachment can be configured in a loop 46, which can be larger than the sensor itself. In this way, the sensor 20 can be positioned between the loop 46 and the strip 22. The second attachment member 44 includes an electrically conductive element for provides communication of signals from the sensor to the strip portion 32 and, in turn, to the respective connector 24. Each of the sensors 20 in the repositionable sensor set 40 can be repositioned individually on the patient to a desired position after breaking the first attachment while still allowing sensing and communication of the sensed electrical signals. For instance, the floating sensor can be moved out away from the strip portion 32 (*e.g.,* up to about 2 inches) depending on the length of attachment member 44 to accommodate defibrillator patches. Alternatively, the sensors 20 in the repositionable set 40 (as well as any sensors) can be removed by cutting them from the sensor section 14.

FIGS. 4 and 5 demonstrate use of the front sensor array section 14 from FIG. 1 positioned on a patient's front torso 50. The sensor array section 14 is positioned on the torso 50 such that the connectors 24 are positioned on lower part of the torso and are arranged pointing to the patient's left side, such as to facilitate their connection to a connector box. In FIG. 4, the sensors 20 in the repositionable section 40 remain connected to the strips 22 via both the first and second attachment members 42 and 44. In FIG. 5, the first attachments 42 have been broken (*e.g.,* by cutting or tearing) and the respective sensors 20 in the repositionable section 40 have been moved to allow space for placement of one or more pads, demonstrated in dotted lines at 52. For instance, the sensor array section 14 can be used during an electrophysiology study during which defibrillator patches are positioned on the patient's torso 50 in the antero-apical position. Thus, by allowing the set of sensors 20 in section 40 to be repositionable, the section 14 can easily accommodate such pads or patches during a data acquisition process.

FIG. 6 depicts an enlarged view of an embodiment of a right-side sensor array section 16. The right-side sensor array section 16, as well as the other sections 12 and 18 are similar in configuration to the front-side array 14 as each include sensors 20 extending from strips 22 having respective strip portions 32 and 34. Adjacent strip portions 32 and 34 that form a respective strip 22 can be connected together by spaced apart connecting elements or tabs 36. Additionally, sensors 20 facing each other along adjacent pairs of strips 22 can be connected together by elongated connecting elements 38. Reference to the description of FIGS. 1, 2 and 3 can be made for additional information about such common features for each of the sensor array sections.

In the example of FIG. 6, each of the sensors 20 is uniquely identified (*e.g.,* labeled with numbers '65' to '127'). The connectors 24 of the sensor array section 16 are labeled R1, R2 and R3, wherein connector R1 includes two columns of sensors identified as sensors labeled '65' to '87', connector R2 includes two columns of sensors labeled '88' to '105', and connector R3 includes two columns of sensors labeled as '106' to '127'. Each of the sensors 20 is electrically connected (via conductors in the strips 22) with terminals on the respective connectors 24 for communicating sensed electrical signals.

The sensor array section 16 is configured for placement on a right-lateral region of a patient's torso (*e.g.,* on the chest generally under the patient's right arm). The sensor array section 16 includes elongated extensions 60 and 62, each of which includes an arrangement of sensors 20 that extend outwardly from a central sensor portion 64 for anterior placement on a patient. Thus, the central portion 64 of sensors is placed over the right lateral region of the patient's chest and the extensions 60 and 62 extend ventral (front) and dorsal (back) for corresponding placement extending in an anterior direction.

In the example of FIG. 6, the strip portion 34 that contains sensors labeled '65' to '77' includes the extension 60 having a set of sensors (*e.g*., the sensors labeled '65' to '68'). The strip portion 34 in the extension 60 has a curved configuration at its end distal the connector R1 that curves with an angle of curvature, indicated at θ, outwardly away from a longitudinal axis extending through a linear portion the strip 34 of the main central portion 64 of the sensor array section 16. For example, the strip portion 34 in the extension 60 and its associated sensors 20 are configured for placement along an upper pectoral (or sub-clavicle) region of a patient's torso. Thus, when the sensor array section 16 is combined with the front section 14, the extension 60 extends in an arc alongside and above the sensors located at top-right-most portion of the front sensor section 14 (see, *e.g.,* FIG. 3).

The strip portion 32 that contains sensors labeled '115' to '127' in the example of FIG. 6 includes the other extension 62 having a set of sensors (*e.g.*, the sensors labeled '115 to '118'). The sensors and strip in this portion are configured to extend along a right, dorsal-anterior region of the patient, such as to terminate at or near the patient's right scapula. Thus, when the sensor array section 16 is combined with the front section 18, the extension 62 and associated sensors extend for positioning alongside adjacent sensors located at top-right-most portion from the back sensor array section 18 and may extend beyond the top sensor in the back section.

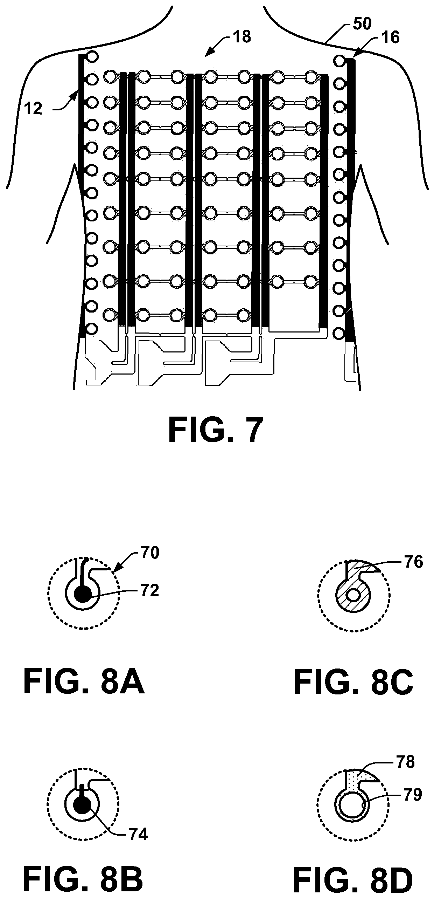

FIG. 7 demonstrates an example of the back sensor array section 18 from FIG. 1 positioned on a back (or dorsal) region of a patient's torso 50. The sensor array section 18 is positioned on the torso 50 such that the connectors 24 are positioned on lower part of the torso and are arranged pointing to the patient's left side, such as to facilitate their connection to a connector box. Also depicted for sake of completeness are end strips and sensors from the left and right sensor array sections 12 and 16, respectively.

FIGS. 8A, 8B, 8C and 8D depict an example embodiment of different layers of a sensor, such as can be utilized in a sensor array system according to an aspect of the invention. In this example, the sensor layers thus can be superimposedly connected in a coaxial arrangement to provide a corresponding sensor. Each sensor 20 in the sensor assembly can be pre-gelled with a conductive paste (*e*.*g*., KCl paste) on top of a contacting surface of a sensor pad as to reduce the skin-sensor interface impedance.

FIG. 8A depicts a contact layer 70 that includes a central body portion 72 of an electrically conductive material, such as silver. An electrically conductive trace can extend from the conductive body portion 72 and along a corresponding strip to terminate at the connector, such as described herein. In FIG. 8B, the respective sensor includes a central portion of a silver and/or silver chloride material that is dimensioned and configured for engaging the conductive central body portion of the layer 70. FIG. 8C depicts a corresponding dielectric layer (*e.g.,* a UV dielectric) 76 can be provided to surround the electrically conductive central portion of FIGS. 8A and 8B. FIG. 8D depicts a soft foam layer (*e.g.,* such as foam commercially available from Vermed Inc. of Bellows Falls, Vermont).

The foam layer 78 includes a central aperture 79 extending through a central portion thereof for exposing the aperture of the dielectric layer 76and the layer 74. The foam layer 78 thus can be dimensioned and configured to be provided over and surround the corresponding dielectric portion 76 (FIG. 8C). Each of these respective layers can be sandwiched together in a coaxial arrangement to provide a corresponding sensor, such as the sensor 56, as well as the sensors in the other embodiments shown and described herein. As described herein, the sensors 20 can be fabricated from materials that are translucent to x-ray and other forms of imaging (*e.g.*, fluoroscopy).

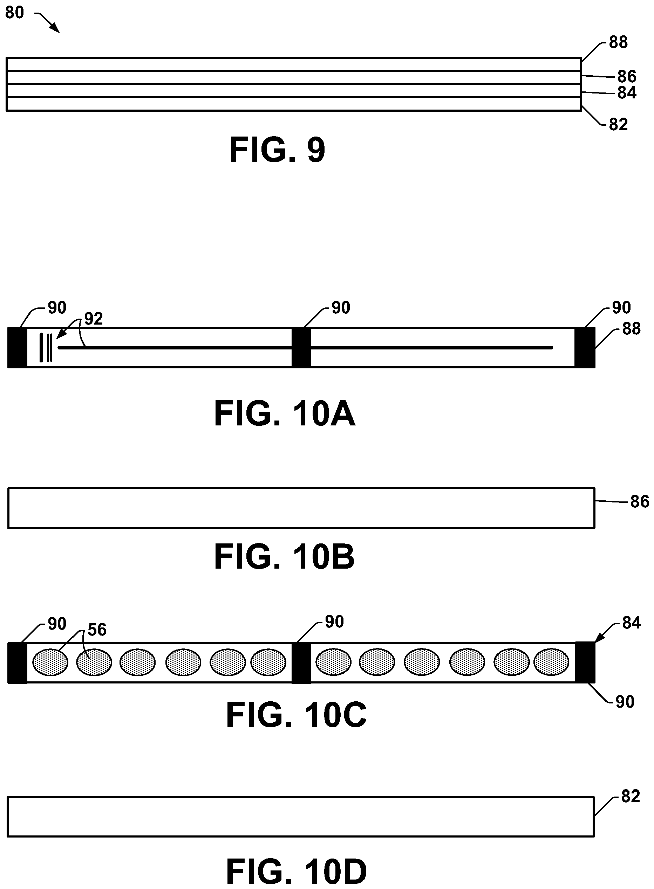

FIG. 9 is a schematic example of a multi-layered substrate strip (or a portion thereof) 80 that can be utilized to implement a strip or a strip assembly. In the example of FIG. 8, four layers 82, 84, 86 and 88 are illustrated as having an exaggerated thickness to demonstrate the existence of multiple layers. The substrate strip 80 is utilized to facilitate recognition and repeated placement of a sensor array assembly. For example, the strip 80 can include ink or other body marking substance that is applied to the skin to facilitate repeated applications of the same or different sensors. The sensors can be applied subsequently and repeatedly based on the markings for acquisition of sensor data with sensors at substantially the same location.

FIGS. 10A, 10B, 10C and 10D are example top views for each of the layers from the strip 80 of FIG. 9 separated from each other. For example, the layer 82 (FIG. 10D) can correspond to a body side peel-off layer that can be utilized to provide protection to the sensor structure such as shown with respect to layer 84 in FIG. 10C. Thus, layer 84 is utilized to contact the body and includes the electrically conductive sensor material forming each of the respective sensors. The shape of the layers can vary according to the configuration of the strips utilized in a given sensor array (see, *e.g.,* the shape of the sensor strips 22 in the respective arrays in FIG. 1).

The sensor layer 84 can also include skin ink markers 90, which in the example of FIG. 10C, are located at the ends of the strip as well as an intermediate portion. Alternatively, the markers 90 can be alignment markers or transparent portions to facilitate alignment with markers already imprinted on the patient's skin. The skin ink in this example is depicted as extending across the strip between side edges thereof. It is to be understood and appreciated that the skin ink can be provided in different locations and in different patterns to facilitate placement of the sensor strips. Additionally, different colored ink can be utilized to indicate different portions of anatomy to which the respective strips can be applied.

Additionally, different skin ink patterns can be utilized on different strips so that if the sensor array or strip or assembly is removed from the patient, the sensor assembly (which may be the same or a different assembly) can be applied in the same position to help ensure that the measurements being obtained will match those previously obtained for a given patient.

The layer 86 (FIG. 10B) can be implemented to provide a body-side peel off for a radio-opaque marker layer 88, such as shown in FIG. 10A. Thus, the layer 88 includes radio-opaque material as markers indicated at 92. In the example of FIG. 10A, the markers are indicated as laterally extending radio-opaque lines between the side edges of the layer and elongated lines extending between the ends. Different numbers and orientations of lines or other markers can be used to differentiate between different sensor strips, such as may be detected using a corresponding imaging modality. In addition to the linear arrangement of substantially parallel radio-opaque markers 92 shown in FIG. 10A, different configurations of radio-opaque material can be provided to facilitate identification and differentiation of each of the respective sensor strips (sensor strips 22 in FIG. 1) after imaging with a suitable modality, such as CT or MRI.

For example, different radio-opaque markers or other types of markers positioned at different locations along the strip can be provided in a predetermined or known pattern to distinctly identify each respective strip. The patterns can be identifiable (*e.g.*, automatically) by employing image processing techniques. Radio-opaque markers can be different shapes, such as circles, triangles, nesters, swirls, spirals and the like, which may be in outlined form or lines that are hollow or the markers may be solid shapes. Additionally, the skin markers that are utilized can also be provided with particular colors or arrangements to identify and indicate a particular type of sensor array assembly or a particular type or arrangement of sensor strip.

In the example of FIG. 9C, skin ink markers 90 may be provided on the contact surface of the marker layer 88. In this way, where the acquisition of sensor location data from an imaging modality is done separately from the acquisition of patient electrical data, the marker layer 90 may be applied to patient during imaging. After imaging, the marker layer 90 can be removed for each part of the sensor assembly, such that the remaining skin ink marks locations of the different parts of sensor assembly. The skin ink can be used to align (at a different, later time) the sensor layer of the sensor assembly in substantially the exact same locations as the marker layer was positioned. As a result, the collection of data in the image acquisition phase and the sensor electrical data acquisition phase can match.

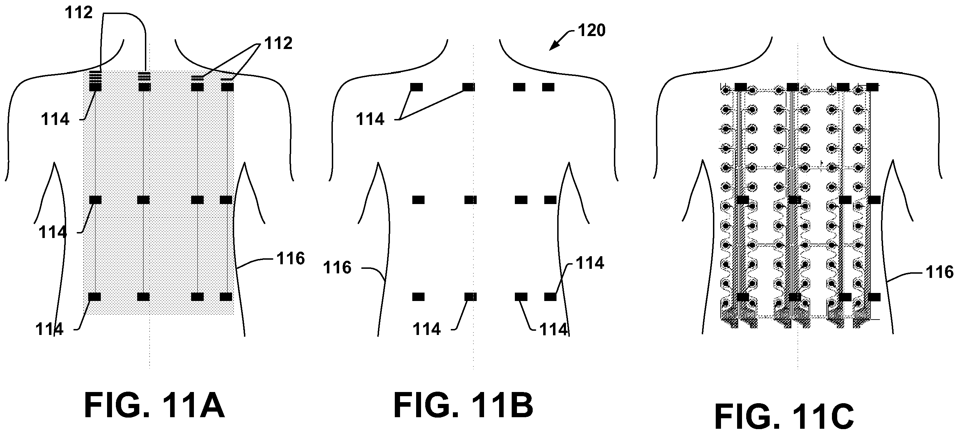

FIGS. 10A, 10B and 10C depict a functional representation of how different layers of the sensor assembly from FIGS. 9A, 9B, 9C and 9D can be utilized, such as for use in detecting respective sensors, as well as ensuring proper replacement of sensors arrays, if necessary. In FIG. 11A, a first pictorial representation 110 demonstrates an arrangement of radio-opaque markers 112 and skin markers 114, such as would be arranged along the length of a sensor array positioned on a patient's torso, such as pictorially depicted at 116. For example, a sheet of material that includes the radio-opaque markers and skin markers are positioned on the patient's torso, such as corresponding to the maker layer 88 of FIG. 10A. Skin markers can be transferred to the patient's skin as the marker layer is applied at spaced-apart locations for each corresponding strip of the sensor assembly.

Different radio-opaque markers 112 can be provided at predetermined locations such as corresponding to and identifying linear axes associated with where sensors are to be positioned. Since the radio-opaque markers 112 have a predetermined spatial relationship with respect to corresponding sensors that are positioned in overlying relationship to the corresponding skin markers 116, locations of each of the respective sensors can be easily determined relative to the detected positions obtained from images while the respective radio-opaque markers are positioned. That is, by placing the radio-opaque markers at different locations along the patient's torso and by using different radio-opaque markers, the location of the sensors can be computed from the image data. Alternatively, as described herein, the imaging can be performed while the sensor array assembly (or a subset of one or more sections thereof) is applied to the patient.

As mentioned above, the marker layer includes radio-opaque markers 112 that are identifiable and segmentable from the image data that is acquired. This layer also includes the skin markers. Referring to pictorial 120 in FIG. 11B, after the marker layer (see, *e.g.,* FIG. 10A) has been removed from the patient's torso, which can be provided on the front, back and sides, corresponding to the locations at which sensor array assemblies are provided for a given patient, skin ink markers will remain on the patient's torso. Thus, in pictorial 120, an arrangement of skin markers 114 is shown corresponding to the location and placement of the transferrable marker layer in pictorial 110 of FIG. 11A.

Additionally, each of the skin markers may be provided with different colors or shapes to facilitate subsequent placement of the sensor array as shown in pictorial 122 in FIG. 11C. Thus, when positioning the sensor array, the sensor array has suitable visual markers that can be aligned with each of the respective skin markers that were imprinted on the patient's body, such as from pictorial 120 (FIG. 11B). By aligning the skin markers on the patient's skin with the corresponding alignment marks in the sensor array section, the position of the respective sensors from the sensor array can be appropriately positioned and mapped into the segmented imaging data according to the radio-opaque markers that were detected. After the sensor array has been positioned on the patient's torso, electrical data can be obtained from the sensors and utilized in performing analysis. While the marking substance that remains on the skin has been described as an ink, those skilled in the art will appreciate various substances that can be utilized for coloring a surface and different designs (e.g., image, text or combinations thereof) that can be used.

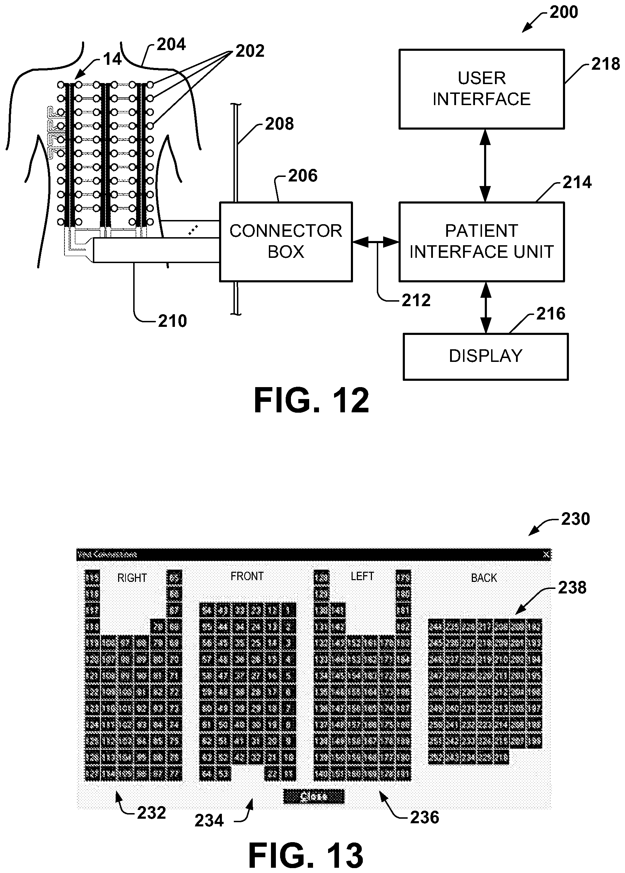

FIG. 12 depicts an example of a sensor data acquisition system 200 that can be utilized to acquire data from a sensor array section having a plurality of sensors 202 applied to a patient's torso 204, such as shown and described herein. For sake of illustration, the sensor array section and sensors 202 in the example of FIG. 12 are in the form of a sensor array section, corresponding to the array section 14 (see FIGS. 1 and 3-5). It will be appreciated that any number and arrangement of the sensors 20 can be applied based on the teachings herein. For instance, to acquire desired data for body surface mapping or electrocardiographic imaging (ECGI), each of the array sections 12, 14, 16, and 18 (FIG. 1) can be applied to the patient's torso as to completely cover the patient's torso with a generally evenly distributed arrangement of plurality sensors (*e.g*., about 252 sensors).

The connectors 24 from the sensor array can be aggregated into a connector box 206 that is positioned near the patient. As one example, the connector box can be attached to bed rail (or other part of a patient's bed), indicated at 208. In one example, the cables from the sensor assembly flow in a direction toward the left side of the patient. The connector box 206 can aggregate multiple sets of cables 210 from different sensor sections as to provide a single output bus 212 that provides electrical signals from the box to a patient interface unit 214. For instance, the cables can be implemented as including jumper ribbon cables that electrically connect each of the connectors 24 with electrical connections at the connector box 206. A corresponding display 216 may provide a graphical user interface for enabling a user to control the data acquisition process and to ensure that appropriate sensor connections have been made.

The patient interface unit 214 can be programmed to provide various features associated with the sensors and the data acquisition process. As an example, a user can employ a pointing device (*e.g.,* a mouse or touch screen) or other input device (*e.g.,* a keyboard) to interact with the patient interface unit 214. Such interactions can change the graphical and textual information on the display 216. For instance, the user interface can be utilized to change between different sensor views or to enable interaction between multiple views that may be displayed concurrently for different parts of the system.

A user can select a sensor, such as on a torso view, and the patient interface unit 214 will highlight the sensor in another view, such as the sensor layout view, and vice versa. A user can also select a sensor in either view, and drag and drop it (or shift - select or click a "modify" button and then select) in the other view to update the sensor registration information associated with the sensors. Information about each sensor can also be utilized to provide an indication (*e.g.*, a check mark, or a warning sign) about whether or not information acquired from one or more sensor is consistent with expected information. For example, the data acquisition system can be programmed to analyze known strip or sensor layout and information that the user may have entered, such as for "removed" leads. This can be utilized for validation and registration of the sensors prior to (e.g., during a set-up phase) as well as during the data acquisition process.

As an example, FIG. 13 depicts an example of sensor mapping GUI 230 that can be employed to provide a graphical representation of sensors as well as information about their operational status. This GUI 230 can be provided, for example, by selecting a "view sensors" or other user interface element from the data acquisition user interface 216 (FIG. 12). The example of FIG. 13 provides a two-dimensional representation of each of the plurality of sensors, as if each of the sensor array sections (*e.g.*, sensor array sections 12, 14, 16 and 18 of FIG. 1) have been spread across a flat surface. Each of the sensors represented in the sensor mapping interface 230 may be numbered corresponding to the labels of individual sensors, such as described herein with respect to the sensor array sections, to facilitate locating respective sensors. Additionally, separate graphical representations are depicted in the GUI 230 for each of the respective sensor array sections, indicated at 232, 234, 236 and 238.

It is appreciated that other views, such as having the sensors arranged along a three-dimensional representation of a human torso, could also be provided. For instance, a user can rotate a three-dimensional representation of the sensor assembly to provide a view of selected sensors or sensor array sections. Alternatively, as depicted in FIG. 13, the representation may be provided as a two-dimensional representation of the sensors, which can include textual and/or graphical identifiers (*e.g.,* color coded) as to the anatomical position of the respective sensors or sets of sensors.

The sensor mapping GUI 230 can also display status or connection information about individual sensors or cabling to the user performing the data acquisition process, such as can be displayed on the graphical element that is associated with each of the respective sensors. For instance, a bad channel (or channels) can be identified by an automated detection process. The indication of whether a sensor or channel might be functioning outside of expected operating parameters can be visualized by a graphical (*e.g.,* a color code or symbol) and/or text based indication on the sensor mapping GUI 230. Alternatively and additionally, a user can employ an input device to manually mark one or more sensors on the mapping GUI 230, such as sensors that might have been removed or repositioned (*e.g.*, as in the case when a defibrillator patch or other pads may be utilized in combination with the sensor array assembly during the data acquisition process).

Additionally, sensor layout information can be provided to the patient interface unit based on an integrated circuit that is incorporated into the sensor array system that has been applied to the patient. The amount of information may include, for example, information about the types of sensors, the number of sensors, the spatial geometry of the sensors or other information that may be useful to the data acquisition process (*e.g.,* diagnostic information). This information can be provided to the patient interface unit through a separate connection (not shown), which may be wired or wireless.

Returning to FIG. 12, the patient interface unit 214 further can be programmed to provide real-time (or near real-time) visual representation of cardiac electrical data for the patient. Additionally, the analysis and processing can be performed as part of a pre-operative procedure. Alternatively or additionally, the analysis and processing can be performed intraoperatively, such as in conjunction with use of an electrophysiology catheter for the patient.

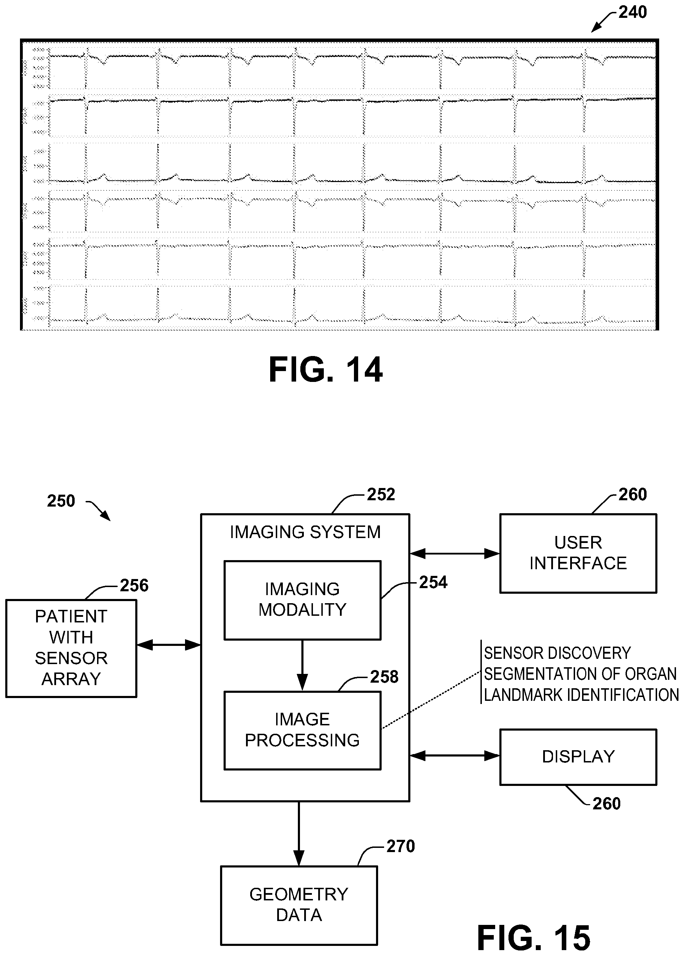

For example, FIG. 14 depicts an example of part of a graphical user interface (GUI) 240 that can be generated and displayed in connection with set up and data acquisition process for a given patient. The GUI 240 enables the user to view electrical data that has been acquired for each sensor on the sensor array assembly. The user can manually, via GUI buttons (not shown), start, stop or otherwise control parameters during the acquisition process. The user can also add markers to the acquired data, such as to identify instances of patient movement or other relevant information during the acquisition process. For instance, the GUI 240 can provide the user with a twelve lead display of the type typically utilized for ECG studies. In this case, the twelve leads corresponding to traditional ECG locations can be automatically selected by the software (*e.g.*, running in a processor of the patient interface unit 214) according to the spatial position of the sensors in the sensor array applied to the patient. Alternatively or additionally, the user can manually (*e.g.*, via a user input device) select the set of sensors that will be used to generate the 12 lead display. The resulting ECG can be graphically displayed to the user, such as depicted in FIG. 14. Other number of channels can also be selected.

Some additional functionality associated with the data acquisition system 200 are as follows:
Data acquisition system 200 can measure ECG electrical activity at the surface of patient's torso at multiple locations, sampling the torso area around the heart from all directions.
Data acquisition system 200 can ensure that electrical signals' transmission from sensor array to the data acquisition station has high fidelity and robustness with respect to EMI/RFT.
Data acquisition system 200 can collect a predefine number (e.g., 1024 or more) potential sample measurements per second, per sensor. All data acquired from the sensors can be synchronized via a time stamp associated with the acquired data.
Data acquisition system 200 can support a sensor ground which will be used as a dynamic amplification baseline potential for all sensors.
Data acquisition system 200 can support a sensor reference, which may be used as a measurement reference.
The data acquisition system can be configured to enable automatic baseline drift correction.
The data acquisition system can enable manual zero correction, *e.g.,* the user can be able to select one or two moments in time, per caliper period, and zero out all potentials at those moments in time, and adjust intermediate potentials accordingly in between.
The data acquisition system can enable automatic detection of bad channels, *e.g.*, channels that have too much noise. These can be displayed or identified to the user via a GUI, such as illustrated in FIG. 13.
The data acquisition system can enable manual declaration to identify ECG channels as being 'bad' per strip.
System 200 can accommodate external ECG lead inputs, and the user can be able to declare the sensor's position.

FIG. 15 depicts an example of image data acquisition and processing system 250 that can be utilized in conjunction with discovering patient anatomy, as well as discovering sensor array layout. The system 250 includes an imaging system 252 that can implement a desired imaging modality 254, such as computed tomography, magnetic resonance imaging, positron emission tomography as well as variants thereof. The imaging system 252 acquires images (*e.g.*, three-dimensional images) of a patient to which one or more sensor array section (*e.g.,* plural sections forming a complete sensor assembly) has been applied prior to the imaging, schematically depicted at 256. Alternatively, as described herein, a marker layer and radio-opaque markers that provide identifiers can be utilized to determine sensor layout and geometry.

The image system 252 can also include an image processing subsystem 258. The image processing 258 can be implemented in conjunction with the imaging modality, such as part of the same system. Alternatively, the image processing 258 can be performed separately (*e.g.*, post-imaging on a computer or workstation) based on the image data acquired from the imaging modality 254. For example, the image processing 258 can be implemented as computer-executable instructions programmed to perform segmentation and identification of anatomical features. Following segmentation of the anatomical image data, the image processing 258 can perform landmark discovery of identifiable heart landmarks. Examples of some landmarks include: aorta, coronary sinus, pulmonary artery, valves (pulmonic valve, aortic valve, tricuspid valve, and mitral valve), posterior septum, anterior septum atrio-ventricular groove, all veins, all arteries and the like.

The image processing 258 is programmed to perform sensor discovery to identify location of each of the sensors in a coordinate system associated with the patient geometry. Mathematical models of the sensors and anatomical geometry can be generated as well. The image processing 258 can generate corresponding geometry data 270, which can include both patient geometry and sensor geometry registered to a common coordinate system.

One or more user interface 260 can be provided for use with implementing the imaging system 250. A user thus can employ the user interface 260 to control selected functionality associated with the imaging system 252 and information that is displayed. A display 260 can be associated with the image system to show anatomical structure and other features, including sensor locations based on the acquired image data and processing that is performed.

In view of the foregoing structural and functional description, those skilled in the art will appreciate that portions of the invention may be embodied as a method, data processing system, or computer program product. Accordingly, these portions of the present invention may take the form of an entirely hardware embodiment, an entirely software embodiment, or an embodiment combining software and hardware. Furthermore, portions of the invention may be a computer program product on a computer-usable storage medium having computer readable program code on the medium. Any suitable computer-readable medium may be utilized including, but not limited to, static and dynamic storage devices, hard disks, optical storage devices, and magnetic storage devices.

Certain embodiments of the invention have also been described herein with reference to block illustrations of methods, systems, and computer program products. It will be understood that blocks of the illustrations, and combinations of blocks in the illustrations, can be implemented by computer-executable instructions. These computer-executable instructions may be provided to one or more processors of a general purpose computer, special purpose computer, or other programmable data processing apparatus (or a combination of devices and circuits) to produce a machine, such that the instructions, which execute via the processor, implement the functions specified in the block or blocks.

These computer-executable instructions may also be stored in computer-readable memory that can direct a computer or other programmable data processing apparatus to function in a particular manner, such that the instructions stored in the computer-readable memory result in an article of manufacture including instructions which implement the function specified in the flowchart block or blocks. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing the functions specified in the flowchart block or blocks.

What have been described above are examples and embodiments of the invention. It is, of course, not possible to describe every conceivable combination of components or methodologies for purposes of describing the invention, but one of ordinary skill in the art will recognize that many further combinations and permutations of the present invention are possible. Accordingly, the invention is intended to embrace all such alterations, modifications and variations that fall within the scope of the appended claims. In the claims, unless otherwise indicated, the article "a" is to refer to "one or more than one."

## Claims

1. A sensor array system (10) comprising:
a plurality of elongated strips (22) of a flexible substrate material that extend from a first end to terminate in a second end spaced therefrom by a length of the substrate material having spaced apart side edges, adjacent pairs of the plurality of elongated strips being interconnected to each other via connecting elements that extend between the adjacent pairs of the plurality of elongated strips to maintain a spatial relationship of the interconnected strips;
a plurality of electrically conductive sensors (20) connected with and distributed along the length of each of the plurality of elongated strips (22) to provide a column of sensors along each respective strip between the first and second ends of each respective strip; and
electrically conductive paths (23) extending from each of the conductive sensors (20) through a respective strip to which it is connected and terminating in a corresponding connector (24) located near the first end of each respective strip (22),
wherein at least one of the plurality of elongated strips (22) comprising a pair of adjacent strip portions (32, 34) connected together and spaced apart from each other along a length thereof by strip connecting elements (36) that form spatial voids (37) between the strip connecting elements (36),
wherein sensors (20) in at least one adjacent pair of the plurality of elongated strips (22) extend away from the respective strip portion toward each other to define a sensor pair, sensors (20) in each sensor pair being connected together by an elongated sensor connecting element (38) extending therebetween, and
wherein at least some of the sensor connecting elements (38) comprise breakable extensions or tabs.

2. The sensor array system of claim 1, wherein the strip connecting elements (36) comprise a frangible portion to facilitate separating and repositioning the adjacent pair of strip portions that the strip connecting elements (36) interconnect.

3. The sensor array system of claim 1, wherein a subset of the plurality of sensors (20) comprise repositionable sensors, at least one attachment member (42, 44) connecting each of the repositionable sensors (20) to a respective strip (32) as to enable repositioning each of the repositionable sensors (20) relative to the respective strip (32).

4. The sensor array system of claim 3, wherein the at least one attachment member further comprises:
a first attachment member (42), which includes no electrically conductive path, connected between a respective one of the repositionable sensors (20) and the respective strip (22) to which the respective one of the repositionable sensors is connected; and
a second attachment member (44) connected between the respective one of the repositionable sensors (20) and the respective strip (22), the second attachment member (44) including an electrically conductive element (46), which forms part of the electrically conductive path for propagating signals from the respective one of the repositionable sensors (20) to its corresponding connector.

5. The sensor array system of claim 4, wherein the second attachment member comprises a length of the flexible substrate material, the second attachment member being longer than the first attachment member.

6. The sensor array system of claim 1 defining a first sensor array section (14), the system further comprising a plurality of other sensor array sections (12, 16, 18), each of the sensor array sections being dimensioned and configured for placement overlying a predetermined region of a patient's torso, and being independently moveable relative to each other, each of the sensor array sections comprising:
a plurality of the elongated strips (22) of the flexible substrate material that extend between first and second ends thereof by a length of the substrate material having spaced apart side edges, each adjacent pair of the elongated strips (22) being interconnected to each other via connecting elements (36) that between the adjacent pair of the plurality of elongated strips (22);
a plurality of the electrically conductive sensors (20) distributed along the length of each of the plurality of elongated strips (22), a contact side of each of the conductive sensors being exposed relative a common contact side of the elongated strips; and
separate electrically conductive paths (23) extending from each of the conductive sensors (20) through a respective strip to terminate in a corresponding connector (24) located near the first end of the respective strip along which the respective sensor is distributed.

7. The sensor array system of claim 6, wherein the sensor array sections comprises:
a first sensor array section (14) configured for placement on a front portion of the patient's torso;
a second sensor array section (12) configured for placement on a left-side portion of the patient's torso;
a third sensor array section (18) configured for placement on a back portion of the patient's torso; and
a fourth sensor array section (16) configured for placement on a right portion of the patient's torso;
wherein the plurality sensor array sections, when placed on the patient's torso, provide a generally evenly distributed arrangement of sensors that circumscribe the patient's torso.

8. The sensor array system of claim 1, wherein at least one of the plurality of elongated strips is longer than other of the elongated strips.

9. The sensor array system of claim 1, wherein at least one of the strips comprises an outwardly curved portion (60) at an end thereof that is opposite the end that is attached to its corresponding connector, and another portion (62) of the at least one strip is substantially linear along the length thereof.

10. The sensor array system of claim 1 in combination with a data acquisition system (200), the data acquisition system comprising a patient interface unit (214) coupled to receive and process electrical signals from at least a substantial portion of the plurality of sensors in the sensor array system.

## Patentansprüche

1. Sensorarraysystem (10), umfassend:
mehrere längliche Streifen (22) eines flexiblen Substratmaterials, die sich von einem ersten Ende erstrecken, um in einem zweiten Ende zu enden, das von einer Länge des Substratmaterials mit beabstandeten Seitenkanten beabstandet ist, benachbarte Paare der mehreren länglichen Streifen, die miteinander über Verbindungselemente verbunden sind, die sich zwischen den benachbarten Paaren der mehreren länglichen Streifen erstrecken, um eine räumliche Beziehung der miteinander verbundenen Streifen aufrechtzuerhalten;
mehrere elektrisch leitende Sensoren (20), die mit der Länge jedes der mehreren länglichen Streifen (22) verbunden sind und über diese verteilt sind, um eine Reihe von Sensoren entlang jedes jeweiligen Streifens bereitzustellen; und
elektrisch leitende Pfade (23), die sich von jedem der leitenden Sensoren (20) durch einen entsprechenden Streifen erstrecken, mit dem sie verbunden sind und der in einem entsprechenden Verbinder (24) endet, der in der Nähe des ersten Endes des jeweiligen Streifens (22) angeordnet ist,
wobei mindestens einer der mehreren länglichen Streifen (22) ein Paar von benachbarten Streifenabschnitten (32, 34), die miteinander verbunden und durch Streifenverbindungselemente (36), die räumliche Hohlräume (37) zwischen den Streifenverbindungselementen (36) bilden, entlang einer Länge davon voneinander beabstandet sind,
wobei Sensoren (20) in mindestens einem benachbarten Paar der mehreren länglichen Streifen (22) sich vom jeweiligen Streifenabschnitt zueinander erstrecken, um ein Sensorpaar zu definieren, wobei Sensoren (20) in jedem Sensorpaar durch ein sich dazwischen erstreckendes längliches Sensorverbindungselement (38) miteinander verbunden sind, und
wobei mindestens einige der Sensorverbindungselemente (38) brechbare Verlängerungen oder Laschen umfassen.

2. Sensorarraysystem nach Anspruch 1, wobei die Streifenverbindungselemente (36) einen zerbrechbaren Abschnitt umfassen, um das Trennen und Umpositionieren des benachbarten Paars von Streifenabschnitten, die die Streifenverbindungselemente miteinander verbinden, zu erleichtern.

3. Sensorarraysystem nach Anspruch 1, wobei eine Teilmenge der mehreren Sensoren (20) umpositionierbare Sensoren umfasst, wobei mindestens ein Befestigungselement (42, 44) jeden der umpositionierbaren Sensoren (20) mit einem entsprechenden Streifen (32) verbindet, um ein Umpositionieren jedes der umpositionierbaren Sensoren (20) im Verhältnis zum jeweiligen Streifen (32) zu ermöglichen.

4. Sensorarraysystem nach Anspruch 3, wobei das mindestens eine Befestigungselement ferner umfasst:
ein erstes Befestigungselement (42), das zwischen einem jeweiligen der umpositionierbaren Sensoren (20) und dem jeweiligen Streifen (22), mit dem der jeweilige der beiden umpositionierbaren Sensoren verbunden ist, keinen elektrisch leitenden Pfad aufweist; und
ein zweites Befestigungselement (44), das zwischen dem jeweiligen der umpositionierbaren Sensoren (20) und dem jeweiligen Streifen (22) verbunden ist, wobei das zweite Befestigungselement (44) ein elektrisch leitfähiges Element (46) umfasst, das einen Teil des elektrisch leitenden Pfads zum Ausbreiten von Signalen von dem jeweiligen der umpositionierbaren Sensoren (20) zu seinem entsprechenden Verbinder bildet.

5. Sensorarraysystem nach Anspruch 4, wobei das zweite Befestigungselement eine Länge des flexiblen Substratmaterials umfasst, das zweite Befestigungselement länger ist als das erste Befestigungselement.

6. Sensorarraysystem nach Anspruch 1, das einen ersten Sensorarrayabschnitt (14) definiert, wobei das System ferner mehrere andere Sensorarrayabschnitte (12, 16, 18) umfasst, wobei jeder der Sensorarrayabschnitte bemessen und konfiguriert ist, um über einem im Voraus bestimmten Bereich des Rumpfes eines Patienten liegend platziert zu werden, und im Verhältnis zueinander unabhängig bewegbar zu sein, wobei jeder der Sensorarrayabschnitte aufweist:
mehrere längliche Streifen (22) des flexiblen Substratmaterials, die sich zwischen ersten und zweiten Enden um eine Länge des Substratmaterials mit voneinander beabstandeten Seitenkanten erstrecken, wobei jedes benachbarte Paar der länglichen Streifen (22) über Verbindungselemente (36) zwischen dem benachbarten Paar der mehreren länglichen Streifen (22) miteinander verbunden ist;
mehrere elektrisch leitfähige Sensoren (20), die entlang der Länge jedes der mehreren länglichen Streifen (22) verteilt sind, wobei eine Kontaktseite jedes der leitfähigen Sensoren im Verhältnis zu einer gemeinsamen Kontaktseite der länglichen Streifen freigelegt ist; und
separate elektrisch leitende Pfade (23), die sich von jedem der leitenden Sensoren (20) durch einen jeweiligen Streifen erstrecken, um in einem entsprechenden Verbinder (24) zu enden, der nahe dem ersten Ende des jeweiligen Streifens angeordnet ist, entlang dem der jeweilige Sensor verteilt ist.

7. Sensorarraysystem nach Anspruch 6, wobei die Sensorarrayabschnitte umfassen:
einen ersten Sensorarrayabschnitt (14), konfiguriert für Platzieren auf einem vorderen Abschnitt des Rumpfes des Patienten;
einen zweiten Sensorarrayabschnitt (12), konfiguriert für Platzieren auf einem linksseitigen Abschnitt des Rumpfes des Patienten;
einen dritten Sensorarrayabschnitt (18), konfiguriert für Platzieren auf einem hinteren Abschnitt des Rumpfes des Patienten; und
einen vierten Sensorarrayabschnitt (16), konfiguriert für Platzieren auf einem rechten Abschnitt des Rumpfes des Patienten;
wobei die mehreren Sensorarrayabschnitte, wenn sie auf dem Rumpf des Patienten angeordnet sind, eine im Allgemeinen gleichmäßig verteilte Anordnung von Sensoren bereitstellen, die den Rumpf des Patienten umgrenzen.

8. Sensorarraysystem nach Anspruch 1, wobei mindestens einer der mehreren länglichen Streifen länger ist als andere der länglichen Streifen.

9. Sensorarraysystem nach Anspruch 1, wobei mindestens einer der Streifen einen nach außen gekrümmten Abschnitt (60) an einem Ende davon aufweist, das dem Ende gegenüberliegt, das an seinem entsprechenden Verbinder befestigt ist, und ein anderer Abschnitt (62) des mindestens einen Streifens im Wesentlichen linear entlang der Länge davon verläuft.

10. Sensorarraysystem nach Anspruch 1 in Kombination mit einem Datenerfassungssystem (200), wobei das Datenerfassungssystem eine Patientenschnittstelleneinheit (214) umfasst, die zum Empfangen und Verarbeiten elektrischer Signale von mindestens einem wesentlichen Teil der mehreren Sensoren im Sensorarraysystem verbunden ist.

## Revendications

1. Système de réseau de capteurs (10) qui comprend :
une pluralité de bandes allongées (22) d'un matériau de substrat souple qui s'étendent d'une première extrémité pour se terminer à une deuxième extrémité espacée de celle-ci d'une longueur du matériau de substrat ayant des bords latéraux espacés, dans lequel les paires adjacentes de la pluralité de bandes allongées sont interconnectées les unes aux autres par l'intermédiaire d'éléments de liaison qui s'étendent entre les paires adjacentes de la pluralité de bandes allongées pour maintenir une relation spatiale des bandes interconnectées ;
une pluralité de capteurs électriquement conducteurs (20) reliés à la pluralité de bandes allongées (22) et répartis le long de la longueur de chacune de celles-ci pour obtenir une colonne de capteurs le long de chaque bande respective entre les première et deuxième extrémités de chaque bande respective ; et
des trajets électriquement conducteurs (23) qui s'étendent de chacun des capteurs conducteurs (20) à travers une bande respective à laquelle chacun est relié et se terminent dans un connecteur (24) correspondant situé à proximité de la première extrémité de chaque bande (22) respective,
dans lequel au moins l'une de la pluralité de bandes allongées (22) comprend une paire de parties de bande (32, 34) adjacentes reliées l'une à l'autre et espacées l'une de l'autre le long d'une longueur de celles-ci par des éléments de liaison de bande (36) qui forment des vides spatiaux (37) entre les éléments de liaison de bande (36),
dans lequel les capteurs (20) dans au moins une paire adjacente de la pluralité de bandes allongées (22) s'étendent hors de la partie de bande respective l'un vers l'autre pour définir une paire de capteurs, dans lequel les capteurs (20) dans chaque paire de capteurs sont reliés l'un à l'autre par un élément de liaison de capteurs allongé (38) qui s'étend entre eux, et
dans lequel au moins certains des éléments de liaison de capteurs (38) comprennent des extensions ou des languettes cassables.

2. Système de réseau de capteurs selon la revendication 1, dans lequel les éléments de liaison de bande (36) comprennent une partie cassable pour faciliter la séparation et le repositionnement de la paire adjacente de parties de bande que les éléments de liaison de bande (36) interconnectent.

3. Système de réseau de capteurs selon la revendication 1, dans lequel un sous-ensemble de la pluralité de capteurs (20) comprend des capteurs repositionnables, au moins un élément de fixation (42, 44) qui relie chacun des capteurs repositionnables (20) à une bande (32) respective de manière à permettre le repositionnement de chacun des capteurs repositionnables (20) par rapport à la bande (32) respective.

4. Système de réseau de capteurs selon la revendication 3, dans lequel ledit au moins un élément de fixation comprend en outre :
un premier élément de fixation (42), qui ne comprend pas de trajet électriquement conducteur, relié entre l'un respectif des capteurs repositionnables (20) et la bande (22) respective à laquelle le capteur respectif des capteurs repositionnables est relié ; et
un deuxième élément de fixation (44) relié entre l'un respectif des capteurs repositionnables (20) et la bande (22) respective, dans lequel le deuxième élément de fixation (44) comprend un élément électriquement conducteur (46), qui fait partie du trajet électriquement conducteur pour propager les signaux du capteur respectif des capteurs repositionnables (20) vers son connecteur correspondant.

5. Système de réseau de capteurs selon la revendication 4, dans lequel le deuxième élément de fixation comprend une longueur du matériau de substrat souple, dans lequel le deuxième élément de fixation est plus long que le premier élément de fixation.

6. Système de réseau de capteurs selon la revendication 1 qui définit une première section de réseau de capteurs (14), dans lequel le système comprend en outre une pluralité d'autres sections de réseau de capteurs (12, 16, 18), dans lequel chacune des sections de réseau de capteurs est dimensionnée et configurée pour être placée de manière à recouvrir une région prédéterminée du torse d'un patient, et peut être déplacée de manière indépendante par rapport aux autres, dans lequel chacune des sections de réseau de capteurs comprend :
une pluralité des bandes allongées (22) du matériau de substrat souple qui s'étendent entre les première et deuxième extrémités de celui-ci d'une longueur du matériau de substrat ayant des bords latéraux espacés, dans lequel toutes les paires adjacentes des bandes allongées (22) sont interconnectées les unes aux autres par l'intermédiaire d'éléments de liaison (36) qui s'étendent entre les paires adjacentes de la pluralité de bandes allongées (22) ;
une pluralité de capteurs électriquement conducteurs (20) répartis le long de la longueur de chacune de la pluralité de bandes allongées (22), dans lequel un côté de contact de chacun des capteurs conducteurs est exposé par rapport à un côté de contact commun des bandes allongées ; et
des trajets électriquement conducteurs séparés (23) qui s'étendent de chacun des capteurs conducteurs (20) à travers une bande respective pour se terminer dans un connecteur (24) correspondant situé à proximité de la première extrémité de la bande respective le long de laquelle le capteur respectif est réparti.

7. Système de réseau de capteurs selon la revendication 6, dans lequel les sections de réseau de capteurs comprennent :
une première section de réseau de capteurs (14) configurée pour être placée sur une partie avant du torse du patient ;
une deuxième section de réseau de capteurs (12) configurée pour être placée sur une partie gauche du torse du patient ;
une troisième section de réseau de capteurs (18) configurée pour être placée sur une partie arrière du torse du patient ; et
une quatrième section de réseau de capteurs (16) configurée pour être placée sur une partie droite du torse du patient ;
dans lequel la pluralité de sections de réseau de capteurs, lorsqu'elles sont placées sur le torse du patient, réalisent un agencement de capteurs répartis généralement uniformément qui circonscrivent le torse du patient.

8. Système de réseau de capteurs selon la revendication 1, dans lequel au moins l'une de la pluralité de bandes allongées est plus longue que les autres des bandes allongées.

9. Système de réseau de capteurs selon la revendication 1, dans lequel au moins l'une des bandes comprend une partie incurvée vers l'extérieur (60) à une extrémité de celle-ci qui est opposée à l'extrémité qui est attachée à son connecteur correspondant, et une autre partie (62) de ladite au moins une bande est sensiblement linéaire le long de la longueur de celle-ci.

10. Système de réseau de capteurs selon la revendication 1 en combinaison avec un système d'acquisition de données (200), dans lequel le système d'acquisition de données comprend une unité d'interface de patient (214) couplée pour recevoir et traiter les signaux électriques qui proviennent d'au moins une grande partie de la pluralité de capteurs dans le système de réseau de capteurs.
